# EUROPÄISCHE PATENTSCHRIFT

(11) **EP 2 065 360 B1**
(45) Veröffentlichungstag und Bekanntmachung des Hinweises auf die Patenterteilung: **19.06.2013**
(21) Anmeldenummer: 09154787.7
(22) Anmeldetag: 29.04.2005
(51) Int. Cl.: C11B 9/00, A61K 8/37, A61Q 13/00

(54) **Verwendung von 3-Methylbenzylisobutyrat als Riechstoff**
Use of 3-methylbenzyl isobutyrate as a fragrance compound
Utilisation de l'isobutyrate de 3-méthylbenzyle en tant qu'ingrédient parfumant

(30) Priorität: 12.05.2004 DE 102004023346
(43) Veröffentlichungstag der Anmeldung: 03.06.2009
(62) Teilanmeldung aus: 05738065.1
(73) Patentinhaber: Symrise AG, 37603 Holzminden (DE)
(72) Erfinder: Dilk, Erich, 37603, Holzminden (DE); Surburg, Horst, 37603, Holzminden (DE)
(74) Vertreter: Eisenführ, Speiser & Partner

(56) Entgegenhaltungen:
- EP-A- 0 056 500
- EP-A- 0 217 159
- STEFFEN ARCTANDER: "Perfume and Flavor Chemicals", 1969, SELBSTVERLAG, MONTCLAIR, N.J. (USA), XP002336565, * Eintrag 1922: Meta-methyl benzyl propionate. *
- STEFFEN ARCTANDER: "Perfume and Flavor Chemicals", 1969, SELBSTVERLAG, MONTCLAIR, N.J. (USA), XP002336566, * Eintrag 1915: Methyl benzyl acetate *
- STEFFEN ARCTANDER: "Perfume and Flavor Chemicals", 1969, SELBSTVERLAG, MONTCLAIR, N.J. (USA), XP002336567, * Eintrag 294: Benzyl-iso-butyrate *

## Beschreibung

Die vorliegende Erfindung betrifft insbesondere die Verwendung von 3-Methylbenzylisobutyrat als Riechstoff.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Duftstoffe bzw. Parfüms mit interessanten Duftnoten darstellen, besteht auch weiterhin ein Bedürfnis nach neuen Verbindungen mit wertvollen Riechstoffqualitäten. Gesucht sind insbesondere neue Riechstoffe, die über ihre geruchlichen Eigenschaften hinaus zusätzliche positive Sekundäreigenschaften, wie z.B. höhere Stabilität, höhere Ausgiebigkeit, besseres Haftungsvermögen, Boosterwirkung usw. aufweisen.

Es war daher die Aufgabe der vorliegenden Erfindung, einen neuen Riechstoff anzugeben, der hervorragende geruchliche Eigenschaften besitzt und mit einer Vielzahl weiterer Riechstoffe stabil kombinierbar ist. Vorzugsweise sollte der anzugebende Riechstoff zur Kombination mit Maiglöckchen-Riechstoffen geeignet und vorteilhafterweise dazu geeignet sein, in Kombination mit diesen Maiglöckchenriechstoffen den Eindruck einer blumigen Frische zu vermitteln.

Diese Aufgabe wird erfindungsgemäß gelöst durch die Verwendung der eingangs bereits angegebenen Verbindung 3-Methylbenzylisobutyrat. Diese Verbindung besitzt überraschenderweise grün-apfelige Geruchseigenschaften, die sich besonders gut zur Erzielung von grün-fruchtigen Kopfnoten in Parfümölmischungen eignen, wie sie häufig gewünscht sind.

Aus dem Stand der Technik ist der Geruch des erfindungsgemäß zu verwendenden Esters nicht bekannt. Aus eigenen Untersuchungen mit strukturell nahe liegenden Verbindungen resultieren folgende Geruchsbeschreibungen.

Die strukturell nächstliegenden Verbindungen 2-Methylbenzylisobutyrat und 4-Methylbenzylisobutyrat wurden insgesamt als geruchlich und für die Parfümerie uninteressant beurteilt. Im Vergleich mit der erfindungsgemäß zu verwendenden Verbindung 3-Methylbenzylisobutyrat überraschend wurden hinsichtlich beider Verbindungen keine fruchtigen Noten festgestellt. Die Geruchsbeschreibungen beider Verbindungen lauten wie folgt:
2-Methylbenzylisobutyrat: fettig, Linalool, Carbinol
4-Methylbenzylisobutyrat: fettig, Anis, Heliotrop.

Das bereits aus der Literatur bekannte 4-Isopropylbenzylisobutyrat verfügt lediglich über einen wässrigen, cuminigen, krautigen Geruch mit Aspekten von Anis.

Das ebenfalls bereits aus der Literatur bekannte 4-Methoxybenzylisobutyrat weist einen ausgeprägten Geruch nach Anis auf, und das 2-Methoxybenzylisobutyrat riecht nach Zimt und Guajak.

In S. Arctander, Perfume and Flavor Chemicals, Vol. I und II, Montclair, N. J., 1969, Selbstverlag wird für den Benzylisobuttersäureester bzw. meta-Methylbenzylpropionat ein fruchtig-blumiger Duft bzw. ein blumiger, leicht fruchtiger Duft moderater Stärke beschrieben. A. Müller, Parfümeur, 2, 43-44, 1928 beschreibt den Duft dieses Esters mit fruchtig, im Unterton nach Kümmel.

EP 0 056 500 A2 beschreibt 3-Methylbenzylpivalat als Riechstoff.

Der erfindungsgemäß zu verwendende Ester 3-Methylbenzylisobutyrat besitzt somit Geruchseigenschaften, die nicht vorhersehbar waren.

Eine hierin im Zusammenhang mit der vorliegenden Erfindung beschriebene Riechstoffmischung umfasst sensorisch wirksame Mengen von 3-Methylbenzylisobutyrat und zumindest einem weiteren Riechstoff. Das Gewichtsverhältnis von 3-Methylbenzylisobutyrat zu den weiteren Riechstoffen sollte vorzugsweise im Bereich von 1:1000 bis 1:5 liegen.

Vorteilhafterweise vermittelt dieser zumindest eine weitere Riechstoff einen Maiglöckchengeruch. Diese Kombination aus 3-Methylbenzylisobutyrat und einem Riechstoff mit Maiglöckchengeruch ist besonders bevorzugt, da das 3-Methylbenzylisobutyrat überraschenderweise bereits in geringer Konzentration den Eindruck einer abrundenden blumigen Frische vermittelt, der neben die grün-apfelige Kopfnote des 3-Methylbenzylisobutyrats und die durch den Maiglöckchenriechstoff vermittelten Geruchseindrücke tritt.

Insbesondere vorteilhaft ist die Kombination von 3-Methylbenzylisobutyrat mit dem Maiglöckchenriechstoff 2,2-Dimethyl-3-(3-methylphenyl)propanol (Majantol ^{®}). Das Gewichtsverhältnis von 3-Methylbenzylisobutyrat zu Majantol liegt dabei vorzugsweise im Bereich von 1:1000 bis 1:5, vorzugsweise 1:100 bis 1:5, weiter vorzugsweise 1:50 bis 1:5.

Die Kombination des 3-Methylbenzylisobutyrat mit Majantol ist aus zwei Gründen besonders bevorzugt. Zum Einen modifizieren bereits geringe Mengen des 3-Methylbenzylisobutyrats die (ohnehin schon hervorragenden) geruchlichen Eigenschaften des Majantol in Richtung blumig-frisch. Und zum Anderen ist es bei geeigneter Verfahrensführung möglich, 3-Methylbenzylisobutyrat und die übliche Vorstufe des Majantol, nämlich 2,2-Dimethyl-3-(3-methylphenyl)-propionaldehyd parallel in einem gemeinsamen Reaktionsansatz herzustellen, siehe dazu unten im Detail. In einer Folgereaktion kann dann selektiv (und ohne vorherige Abtrennung eines der beiden Reaktionsprodukte) der Aldehyd zum Majantol hydriert werden. Das dann resultierende finale Produktgemisch enthält die Riechstoffe 3-Methylbenzylisobutyrat und Majantol.

3-Methylbenzylisobutyrat kann daher (erfindungsgemäß) als Riechstoff verwendet werden, insbesondere zur Vermittlung einer grün-fruchtigen Kopfnote in einer Riechstoffmischung und besonders bevorzugt zum Erzeugen des Eindrucks einer abrundenden blumigen Frische in Kombination mit Maiglöckchenriechstoffen. Über die Verwendung in Riechstoffmischungen hinaus kann 3-Methylbenzylisobutyrat ein sensorisch wertvoller oder sogar der sensorisch entscheidende Bestandteil einer Vielzahl von Produkten sein. Erfindungsgemäß behandelte Produkte umfassen einen Träger oder ein Substrat sowie eine mit dem Träger bzw. dem Substrat in direktem Kontakt stehende sensorisch wirksame Menge an 3-Methylbenzylisobutyrat.

Bevorzugte erfindungsgemäß zu behandelnde Produkte sind ausgewählt aus der Gruppe bestehend aus: alkoholischen Parfüms, Körperpflegeprodukten und im Haushalt zu verwendenden Reinigungs- oder Pflegeprodukten. Dabei sind die Körperpflegeprodukte vorzugsweise ausgewählt aus der Gruppe bestehend aus Seifen, Duschgelen, Shampoos, Badezusätzen, Hautcremes, Körperlotionen und Deodorantien, und die Reinigungsmittel sind vorzugsweise ausgewählt aus der Gruppe bestehend aus Waschmitteln, Wäscheweichspülern, Raumluftverbesserern und Reinigern.

Das 3-Methylbenzylisobutyrat lässt sich mit anderen Riechstoffen in verschiedenen, unterschiedlichen Mengenverhältnissen zu neuartigen Parfümkompositionen kombinieren.

Beispiele für Riechstoffe, mit denen der erfindungsgemäß zu verwendende Ester vorteilhaft kombiniert werden kann, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001.

Im einzelnen seien genannt:
Extrakte aus natürlichen Rohstoffen wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam ; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;
Einzel-Riechstoffe aus der Gruppe
der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B.
Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B.
Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B.
2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B.; 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B.; 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril;
der aliphatischen Carbonsäuren und deren Ester wie z.B. (E)-und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)-und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z. B.; Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z. B.:; Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on ; 1,3,4,6,7,8a-Hexahydro-1,1,5,5-tetramethyl-2H-2,4a-methanonaphthalen-8(5H)-on; Nootkaton ; Dihydronootkaton ; alpha-Sinensal ; beta-Sinensal ; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. ; 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. ; 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. ; 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B.; 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. ; Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. ; Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. ; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Maiglöckchenriechstoffe, mit denen der erfindungsgemäß zu verwendende Ester vorteilhaft kombiniert werden kann, sind z.B. Hydroxycitronellal, Methoxycitronellal, Cyclamenaldehyd, 1-(4-Isopropyl-cyclohexyl)ethanol (Mugetanol^{®}), 4-tert.-Butyl-α-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)propanol (Majantol^{®}), 3-Methyl-3-(3-methylbenzyl)-butan-2-ol, 2-Isobutyl-4-methyltetrahydro-2H-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5H-inden-5-yliden-butanal (Dupical^{®}), Vernaldehyd, 4-(4-Methyl-3-penten-1-yl)-3-cyclohexencarbaldehyd (Vertomugal^{®}), Octahydro-5-(4-methoxybutyliden)-4,7-methano-1H-inden (Mugoflor^{®}), 2,6-Dimethyl-2-heptanol (Freesiol^{®}), 1-Ethyl-1-methyl-3-phenylpropanol (Phemec^{®}), Profarnesol, Dihydrofarnesol, Farnesol, Hydroxycitronellaldimethylacetal, Tetrahydrolinalool, Ethyllinalool, Hexylbenzoat; geruchlich besonders interessant ist - wie ausgeführt - die Kombination von 3-Methylbenzylisobutyrat und 2,2-Dimethyl-3-(3-methylphenyl)propanol (Majantol^{®}).

In Parfümkompositionen (Riechstoffmischungen) beträgt die eingesetzte Gesamtmenge an erfindungsgemäß zu verwendendem Ester vorteilhafterweise 0,05 bis 50 Gew.-%, vorzugsweise 0,5 bis 20 Gew.-%, bezogen auf die gesamte Parfümöl-Komposition.

In Kombination mit Maiglöckchenriechstoffen wird der erfindungsgemäß zu verwendende Ester vorzugsweise in einer Konzentration von 0,05 bis 10 Gew.-%, bevorzugt 0,5 bis 5 Gew.-%, bezogen auf die Summe der Maiglöckchenriechstoffe, eingesetzt. In diesen Konzentrationen bewirkt der erfindungsgemäß zu verwendende Ester z.B. in Kombination mit Majantol^{®} eine spezifische und charakteristische blumige Frische (siehe auch Beispiel 3 unten).

Den erfindungsgemäß zu verwendenden Ester enthaltende Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können den erfindungsgemäß zu verwendenden Ester enthaltende Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Den erfindungsgemäß zu verwendenden Ester enthaltende Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Den erfindungsgemäß zu verwendenden Ester enthaltende Parfümöle können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Die Herstellung des 3-Methylbenzylisobutyrats kann nach den allgemein üblichen Methoden zur Herstellung von Carbonsäurestern erfolgen, z.B. durch Veresterung von Isobuttersäure mit 3-Methylbenzylalkohol, kommerziell erhältlich bei der Fa. Sigma-Aldrich, Acylierung von 3-Methylbenzylalkohol mit Isobuttersäurechlorid bzw. Isobuttersäureanhydrid oder durch Umesterung von Methyl- bzw. Ethylisobutyrat mit 3-Methylbenzylalkohol.

Besonders bevorzugt ist ein Verfahren zur Herstellung von 3-Methylbenzylisobutyrat, das die folgenden Schritte umfasst:
- Herstellen einer Reaktionsmischung umfassend die Edukte (a) 3-Methylbenzylalkohol und/oder 3-Methylbenzylchlorid sowie (b) Isobuttersäure (2-Methylpropionsäure) und/oder Isobutyrat und/oder Isobutyraldehyd und
- Einstellen von Reaktionsbedingungen in der Reaktionsmischung, bei denen 3-Methylbenzylisobutyrat direkt oder über Zwischenschritte aus den Edukten gebildet wird.

Es versteht sich dabei, dass bei alleiniger Anwesenheit von (b) Isobutyraldehyd zunächst aus diesem Aldehyd die korrespondierende Säure bzw. das korrespondierende Butyrat gebildet wird, bevor dann in einem Folgeschritt die Umsetzung zu 3-Methylbenzylisobutyrat erfolgt. Ein bevorzugtes Verfahren ist eines, in dem die Reaktionsmischung Isobutyraldehyd umfasst, das unter den Reaktionsbedingungen zu Isobutyrat oxidiert oder disproportioniert wird.

Mit Blick auf die bevorzugten Riechstoffmischungen, welche neben 3-Methylbenzylisobutyrat auch Majantol umfassen, ist ein Herstellungsverfahren bevorzugt, bei dem die Reaktionsmischung 3-Methylbenzylchlorid und Isobutyraldehyd (2-Methylpropanal) umfasst und die Reaktionsbedingungen in der Reaktionsmischung eingestellt werden, dass simultan 3-Methylbenzylisobutyrat und 2,2-Dimethyl-3-(3-methylphenyl)-propionaldehyd gebildet werden.

Das gebildete 2,2-Dimethyl-3-(3-methylphenyl)-propionaldehyd (Majantal) kann dann im Wege der Hydrierung oder Reduktion in Majantol überführt werden, beispielsweise so wie es in der WO 03/089394 oder EP 0 217 159 B1 beschrieben ist. Es ist hierbei nicht erforderlich, die erfindungsgemäß zu verwendende Verbindung 3-Methylbenzylisobutyrat vor Durchführung der Hydrierung oder Reduktion abzutrennen. Nach Hydrierung oder Reduktion liegen dann 3-Methylbenzylisobutyrat und Majantol nebeneinander im Produktgemisch vor.

Weitere Aspekte der vorliegenden Erfindung ergeben sich aus den nachfolgenden Beispielen und den beigefügten Patentansprüchen.

### Beispiel 1

3-Methylbenzylisobutyrat
25g (0,2 Mol) 3-Methylbenzylalkohol (Aldrich), 18g (0,2 Mol) Isobuttersäure und 0,6 g p-Toluolsulfonsäure werden in einer 500 mL-Dreihalskolbenapparatur am Wasserabscheider bis zur vollständigen Wasserabtrennung erhitzt. Anschließend wird zunächst mit verdünnter Natriumhydrogencarbonatlösung und danach mit Wasser gewaschen. Nach Abdestillieren des Toluols wird der Rückstand über ein Kugelrohr destilliert. Man erhält 23 g (60% d. Th.) Produkt.

Geruch: Grün, Apfel, Frucht 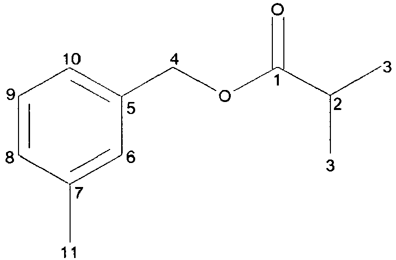 NMR-Spektren (200 MHz, interner Standard: Tetramethylsilan):
¹H-NMR (CDCl₃, ppm) δ 1,19 (d, J=7,0 Hz, 6H), 2,36 (m, J=0,7 Hz, 3H), 2,60 (septett, J=7,0 Hz, 1 H), 5,07 (s, 2H), 7,09-7,31 (m, 4H).
¹³C-NMR (CDCl₃, ppm) δ 18,9 (q), 21,3 (q), 33,9 (d), 66,0 (t), 125,0 (d), 128,3 (d), 128,6 (d), 128,7 (d), 136,1 (s), 138,0 (s), 176,6 (s).
Massenspektrum
m/z (rel. Int.): 27(4), 43(26), 71(13), 77(16), 91(8), 105(100, bp), 122(75), 192(32, M⁺)

### Beispiel 2 (simultane Herstellung von 3-Methylbenzylisobutyrat und 2,2-Dimethyl-3-(3-methylphenyl)-propionaldehyd)

### Reaktionsparameter: Temperatur: 45°C; Molverhältnis Isobutyraldehyd: 3-Methylbenzylchlorid = 1,4:1

120g Natronlauge, 11,8g Tetrabutylammoniumjodid, 180g Wasser und 310g Toluol werden in einem 4L-Doppelmantelgefäß vorgelegt und auf 45°C erwärmt. Bei dieser Temperatur wird innerhalb von 12 Stunden unter Rühren eine Mischung aus 200g Isobutyraldehyd und 290g 3-Methylbenzylchlorid zudosiert. Nach dem Dosierende rührt man 3 Stunden nach. Der Ansatz wird bei 45°C mit 200g Wasser versetzt und 20 Minuten gerührt. Nach Phasentrennung erhält man 566g alkalische Wasserphase und 748g organische Phase.

Die organische Phase enthält entsprechend gaschromatographischer Analyse (Leichtsieder unberücksichtigt) 56,8% 2,2-Dimethyl-3-(3-methylphenyl)-propionaldehyd und 3,8% 3-Methylbenzylisobutyrat. Die organische Phase wird nach Abrotieren des Toluols an einer 30cm Füllkörperkolonne bei 2,1 mbar in einem Temperaturbereich von 54-62°C destilliert. Man erhält 288g einer Hauptfraktion, in der 75% 2,2-Dimethyl-3-(3-methylphenyl)-propionaldehyd und 5,3% 3-Methylbenzylisobutyrat enthalten sind.

### Beispiel 3

### Herstellung eines Parfümöles mit Maiglöckchen-Geruch unter Verwendung von 3-Methylbenzylisobutyrat

Es werden zunächst folgende Komponenten vermischt:

| Riechstoff | Gew.-Teile |
|---|---|
| Methyldihydrojasmonat | 50 |
| Diethylphthalat | 61 |
| Indol | 1 |
| Geraniol | 25 |
| Phenylethylalkohol | 50 |
| I-Citronellol | 55 |
| Hexylzimtaldehyd | 270 |
| cis-/trans-3-Hexenylacetat | 3 |
| Linalool | 45 |
| Linalylacetat | 10 |
| Majantol | 80 |
| Summe | 650 |

Durch Hinzufügen von 3 Gewichtsteilen 3-Methylbenzylisobutyrat erhält der natürliche Maiglöckchen-Geruch der Parfümöl-Komposition eine grün-apfelige Kopfnote. Außerdem resultiert durch den Zusatz von 3-Methylbenzylisobutyrat eine abrundende blumige Frische.

## Patentansprüche

1. Verwendung von 3-Methylbenzylisobutyrat als Riechstoff, insbesondere zur Vermittlung einer grün-fruchtigen Kopfnote in einer Riechstoffmischung.

2. Verfahren zum Vermitteln, Verstärken oder Modifizieren eines Geruches, wobei ein Produkt mit 3-Methylbenzylisobutyrat kontaktiert oder versetzt wird.

## Claims

1. Use of 3-methylbenzyl isobutyrate as a fragrance, in particular to convey a green-fruity top note within a mixture of fragrances.

2. Method for conveying, intensifying or modifying a smell, wherein a product is contacted or mixed with 3-methylbenzyl isobutyrate.

## Revendications

1. Utilisation de l'isobutyrate de 3-méthylbenzyle en tant que substance odoriférante, en particulier pour conférer une note de tête fruitée verte à un mélange de substance odoriférante.

2. Procédé destiné à conférer, à intensifier ou à modifier une odeur, dans lequel un produit est mis en contact avec ou mélangé à l'isobutyrate de 3-méthylbenzyle.
